# FASCICULE DE BREVET EUROPEEN

(11) **EP 0 796 625 B1**
(45) Date de publication et mention de la délivrance du brevet: **03.09.2003**
(21) Numéro de dépôt: 97480004.7
(22) Date de dépôt: 09.02.1997
(51) Int. Cl.: A61L 11/00, B09B 3/00

(54) **Procédé de désinfection thermique de déchets notamment à risques biologiques et dispositif de mise en oeuvre de ce procédé**
Hitzedesinfektion von biologisch kontaminierten Abfällen sowie Vorrichtung zum Durchführen des Verfahrens
Process for thermal disinfection of biologically contaminated waste and device for carrying out the process

(30) Priorité: 09.02.1996 FR 9601828
(43) Date de publication de la demande: 24.09.1997
(73) Titulaire: Golden Harvest S.A., 2636 Luxembourg (LU)
(72) Inventeur: Aubert, Bruno, 30330 Connaux (FR)
(74) Mandataire: Rhein, Alain

(56) Documents cités:
- EP-A- 0 317 047
- EP-A- 0 430 898
- WO-A-90/15419
- FR-A- 2 536 011
- FR-A- 2 715 850
- US-A- 5 447 685
- DATABASE WPI Section Ch, Week 9532 Derwent Publications Ltd., London, GB; Class A17, AN 95-243416 XP002033021 & JP 07 149 401 A (SUZUKI KAKO KK) , 13 juin 1995

## Description

La présente invention a pour objet un procédé de désinfection, notamment de déchets à risques biologiques et le dispositif de mise en oeuvre de ce procédé.

Les désinfections de déchets médicaux connues de l'état de la technique sont les suivantes:
- les déchets peuvent être collectés en vue d'un traitement dans une unité spécialisée, telle qu'un incinérateur ou une installation de traitement thermique (vapeur surchauffée, hautes fréquences, micro-ondes etc...). Toutefois, de multiples manipulations (broyage, tri éventuel) sont nécessaires et un stockage intermédiaire pouvant aller jusqu'à plusieurs jours, conduit à une prolifération inacceptable des germes pathogènes et à des risques de contamination tout le long de la chaîne de traitement (poubelle, broyeur, véhicule transporteur etc ...) ;
- Les déchets peuvent être traités sur place afin d'éliminer le problème de prolifération lors du stockage intermédiaire. Des machines fonctionnant par jets d'ozone ou de solutions désinfectantes sont en cours de développement, mais elles ne permettent pas de désinfecter des objets clos comme des aiguilles de prélèvement dans lesquels les agents désinfectants ne rentrent pas.
- D'autres machines, thermiques cette fois-ci, commencent à être mises en oeuvre, mais celles-ci nécessitent soit un broyage des déchets pour un meilleur transfert thermique, soit de la vapeur surchauffée, soit une source radio-fréquence pour un chauffage plus rapide. Elles s'adressent à des capacités de traitement élevées (20 à 250 kg/h) et représentent un investissement lourd en occupant une place importante conduisant souvent à les mettre loin du lieu de génération des déchets, ce qui repose le problème du stockage intermédiaire et de la prolifération des germes.

Le problème des petites structures médicales comme les médecins praticiens ou les petits laboratoires d'analyses qui ne génèrent qu'une faible quantité de déchets journellement reste donc entier.

En effet, ces petites structures génèrent de 0,1 à 5 kg de déchets par jour et sont contraintes de payer très cher leur transport et leur traitement dans une installation adaptée.

Pour notamment remédier à ces inconvénients, un des buts de la présente invention est de proposer un procédé de désinfection des déchets, qui puisse s'effectuer sur le lieu même de génération des déchets, qui réduise de façon significative la contamination, et qui puisse désinfecter des petites quantités de déchets pour un coût économique et avec un encombrement minimum.

Le procédé selon l'invention est destiné à la désinfection thermique de déchets notamment à risques biologiques, et est caractérisé en ce que ces déchets sont placés dans une zone de compactage, puis soumis à une pression, les gaz émis par le compactage desdits déchets sont filtrés avant d'être évacués, puis le moule est étanchéifié et un chauffage est appliqué en contrôlant simultanément la température et la pression à l'intérieur du moule, la température dudit chauffage étant incluse entre 100 et 200°C, toutes les pièces ayant été en conctact avec les déchets étant également désinfectées. Avantageusement, la température de chauffage est supérieure à 134°C.

Suivant un mode de réalisation préféré de l'invention, les déchets sont préalablement recouverts de polymère, de préférence composite, ou placés dans un sac en polymère, de préférence composite, avant d'être introduits dans la zone de compactage. De préférence, le sac est en polymère composite, la surface extérieure du sac étant en polypropylène ou en polyamide et la surface intérieure du sac étant en polyéthylène.

Suivant un autre mode de réalisation préféré de l'invention, le procédé comprend en outre une étape de démoulage incluant le refroidissement jusqu'à une température inférieure à 60°C de la zone de compactage et du déchet compacté qu'elle contient, le retour de ladite zone à pression atmosphérique et l'évacuation du déchet.

Un autre but de l'invention est de proposer un dispositif de mise en oeuvre de ce procédé comprenant un piston muni d'un vérin, un moule, un filtre, un calorifuge, un sac rempli de déchets à désinfecter, des moyens de chauffage et des moyens d'étanchéifier le dispositif, caractérisé par le fait que le piston est prévu apte à refermer de manière étanche le moule au moyen d'un joint torique ou gonflable, et que les moyens de chauffage sont constitués de résistances électriques et/ou de varistances dont sont munis le moule et le piston.

Suivant un mode de réalisation préféré de l'invention, le dispositif comprend en outre un capteur de pression et/ou une jauge de contrainte et/ou une vanne d'isolement 14 et/ou un extracteur 16 et/ou un trou d'évacuation 15 relié à une vanne d'isolement 12 et/ou un cadre.

De préférence, la face du piston 2 qui est en contact avec les déchets est munie d'ergots 10.

Avantageusement, le vérin 3 est un vérin hydraulique ou un vérin mécanique commandé par un moteur électrique.

Suivant un mode de réalisation préféré de l'invention, les moyens d'étanchéifier le dispositif comprennent des joints torriques ou gonflables astucieusement disposés et/ou des vannes d'isolement 12 et 14.

De préférence, le piston 2, le moule 4 et la réhausse 1 sont totalement ou partiellement élaborés en métal inoxydable ayant une dureté suffisante pour ne pas être rayés ni détériorés lors du compactage d'aiguilles et de scalpels.

De préférence, le piston 2, le moule 4 et la réhausse de guidage 1 sont totalement ou partiellement élaborés ou recouverts en tout métal à haute conductibilité thermique.

Par ailleurs, le cadre peut être muni d'une butée 19.

Suivant un mode de réalisation particulier de l'invention, le filtre 6 est placé de manière à ce que la température de ce filtre et de l'espace se trouvant entre les déchets et ledit filtre 6 soit sensiblement égale à la température de la zone de compactage. Le filtre 6 est préférentiellement un filtre à très haute efficacité tel que notamment une membrane minérale et/ou du charbon actif et/ou un feutre en téflon.

Suivant un autre mode de réalisation particulier de l'invention, le capteur de pression et/ou la jauge de contrainte sont disposés sur le circuit hydraulique du vérin 3.

La description détaillée qui suit se réfère aux figures jointes qui illustrent non limitativement l'invention.

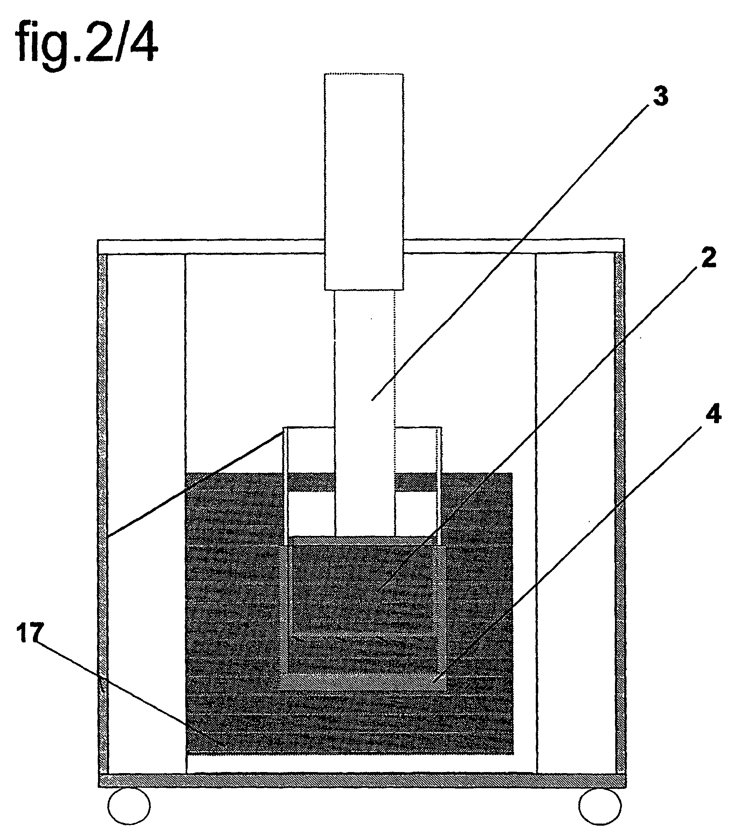

Les figures 1, 2 et 3 montrent des vues en coupe du dispositif selon l'invention.
La figure 1 représente une vue en coupe du dispositif selon l'invention en position ouverte prêt à recevoir un sac de déchets ;
La figure 2 représente une vue en coupe du dispositif selon l'invention en position fermée en cours de traitement ;
La figure 3 représente une vue en coupe du dispositif selon l'invention en position d'évacuation de déchets compactés.

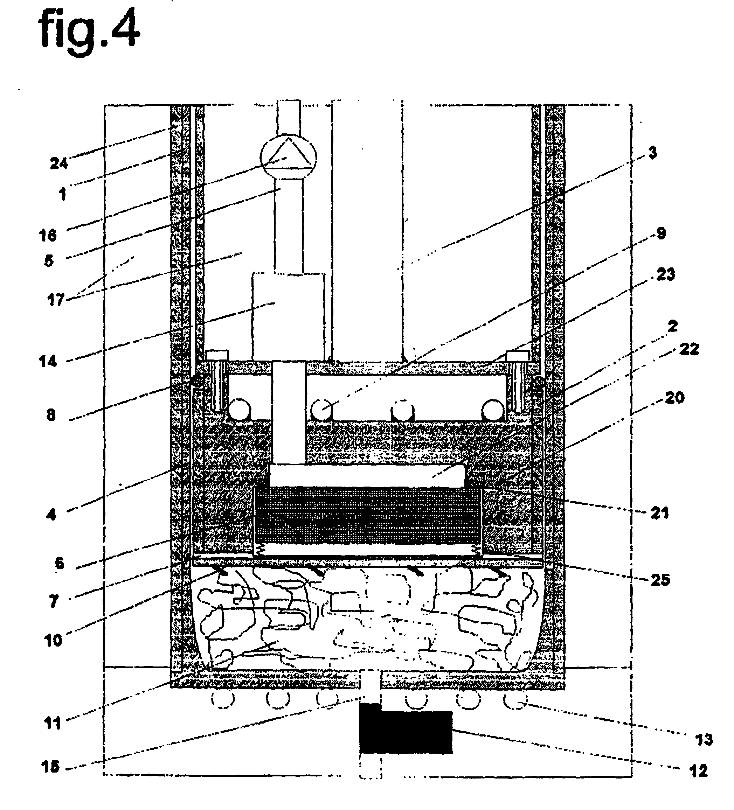

La figure 4 montre une vue en coupe détaillée du piston 2, du moule 4 et de sa réhausse 1 avec lesquels les déchets 11 sont compactés et chauffés.

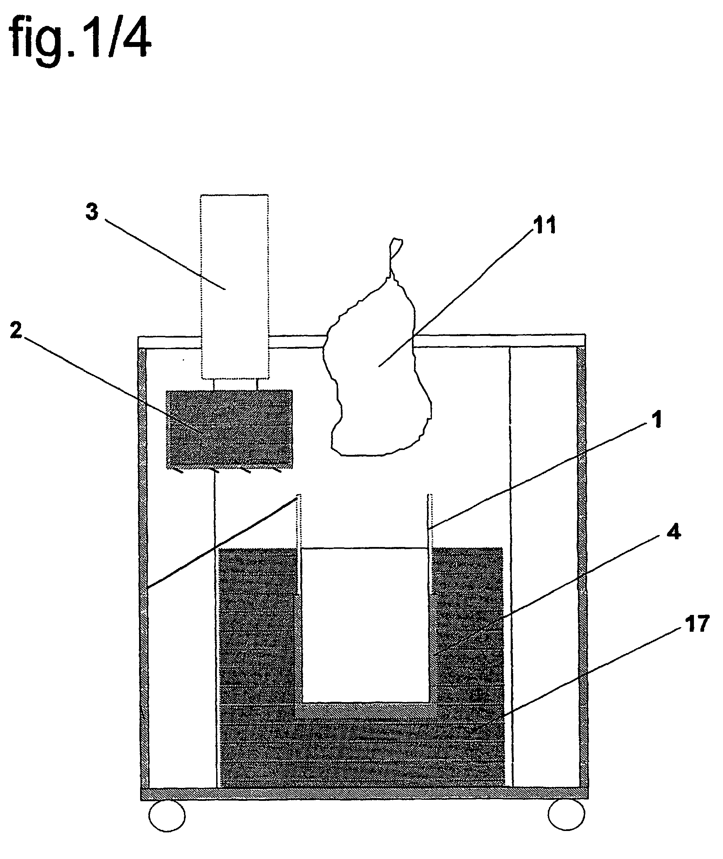

Sur la figure 1 est représenté un dispositif de mise en oeuvre du procédé selon l'invention, comprenant une réhausse 1, un piston 2 muni d'un vérin 3, un moule 4, un calorifuge 17 et un sac 11 rempli de déchets à désinfecter.

Le sac 11 rempli de déchets est introduit dans la zone de compactage. La composition de ce sac 11 est choisie en fonction de la température de traitement à appliquer. De préférence, il s'agit d'un sac en polymère, de préférence composite, de forte épaisseur. Avantageusement, on utilisera un sac en polymère composite tel que la surface extérieure du sac étant en polypropylène ou en polyamide et la surface intérieure du sac étant en polyéthylène.

Le sac 11 de déchets est ensuite introduit dans le moule 4 quand le piston 2 et son vérin 3 sont dégagés. Le piston 2 et le vérin 3 sont ensuite mis en place au-dessus du moule, soit manuellement soit grâce à une motorisation (non représentée). Le vérin 3, qui peut être un vérin hydraulique ou un vérin mécanique commandé par un moteur électrique, peut alors faire descendre le piston 2.

Le piston 2 comprend une partie métallique creuse qui peut être en métal inoxydable, ce qui permet de limiter la corrosion due aux conditions d'emploi (milieu humide chaud et sous pression avec la possibilité de présence de certains acides et/ou bases). De préférence, l'extérieur du piston est dans un métal d'une dureté suffisante pour ne pas être rayé ni détérioré lors du compactage d'aiguilles et de scalpels en métal inoxydable.

Suivant un mode de réalisation particulier de l'invention, l'intérieur du piston est rempli ou recouvert par un métal ayant une très bonne conductibilité thermique, tel que notamment l'aluminium. Cette caractéristique particulière permet d'amener le piston 2, le filtre 6, et l'espace situé entre le sac 11 de déchets, le plus rapidement et de la façon la plus homogène possible, à la température de la zone de compactage. On évite ainsi la formation de gradients de températures qui conduiraient à l'apparition de condensations pendant le cycle de chauffage sur les parties les plus froides, ces condensations étant susceptibles de mouiller le filtre 6 et donc de le dégrader. Le filtre est ainsi désinfecté à chaque traitement, limitant au mieux les risques d'accumulation de contamination bactériologique et/ou virale. De préférence, le filtre n'est pas en contact avec le déchet. Suivant un mode de réalisation particulier de l'invention, le filtre 6 est directement en contact avec la face inférieure du piston.

Suivant un autre mode de réalisation de l'invention, le moule 4 et la réhausse 1 sont en métal inoxydable afin de limiter la corrosion due aux conditions d'emploi (milieu humide chaud et sous pression avec la possibilité de présence de certains acides et/ou de bases), de dureté suffisante pour ne pas être rayé par des aiguilles ou scalpels. De préférence, la rehausse 1 est élaborée en métal conducteur, car une de ses fonctions, outre le guidage, est d'apporter de la chaleur à la partie haute du moule. La rehausse est au contact de la partie supérieure du piston, à proximité d'une ou plusieurs résistances.

Suivant un mode de réalisation particulier de l'invention, l'air est évacué, lors du compactage, vers le filtre et la vanne 14 au moyen de trous 7. Ces trous sont nécessaires pour le passage de l'air, mais ils ont également la fonction de laisser passer l'éventuelle condensation qui pourrait se former, dans le cas où le filtre 6 et la vanne 14 sont dans le piston 2, sur les parois intérieures en aluminium du piston 2.

Le moule 4 et le piston 2 sont munis d'éléments chauffants de type résistances électriques, éventuellement associées à un régulateur de température, tel que notamment un thermostat. Suivant un mode de réalisation préféré de l'invention, la face inférieure du piston en contact avec les déchets est munie d'une ou plusieurs résistances.

Suivant une variante avantageuse de l'invention, ces résistances sont des varistances qui ont une conductibilité électrique telle qu'elles cessent de fonctionner lorsqu'elles ont atteint la température de Curie. Elles s'autorégulent ainsi à une température prédéterminée en fonction de leur composition. Cette disposition permet de faire l'économie d'un régulateur et d'être certain que la température ne s'emballera pas à la suite d'un dysfonctionnement du régulateur.

On prendra avantageusement des varistances qui se limitent à 230°C, ce qui, compte tenu des résistances thermiques de contact, permettra d'assurer une température d'équilibre au niveau de ces derniers de 200°C environ (pour un traitement des déchets à 134°C, on pourra prendre des varistances s'autolimitant à 180°C).On notera que le chauffage des déchets au-delà de 200°C est superflu et pourrait poser des problèmes de décomposition de certains plastiques avec formation de composés toxiques (cyanures). De préférence, la température de chauffage se situe entre 100 et 200°C, et est avantageusement supérieure à 134 °C.

Suivant un mode de réalisation particulier de l'invention, le moule 4 est également muni d'un capteur de pression et/ou une jauge de contrainte (non représenté). Dans le cas où le vérin 3 est un vérin hydraulique, ce capteur de pression et/ou cette jauge de contrainte peut avantageusement être disposé sur le circuit hydraulique du vérin 3. Cette disposition permet de connaître à tout moment la pression du circuit hydraulique et donc celle exercée sur le piston 2 par la pression de vapeur consécutive au chauffage des déchets.

Suivant un mode de réalisation avantageux de l'invention, dans le cas où le vérin 3 est un vérin mécanique commandé par un moteur électrique, une jauge de contrainte est disposée sur le vérin 3. Cette jauge permettra de connaître la pression exercée par ledit piston et est une alternative à l'emploi d'un capteur de pression.

De préférence, le filtre 6 est un filtre à très haute efficacité tel que notamment une membrane minérale et/ou du charbon actif et/ou un feutre en téflon. De préférence, le filtre est un feutre en téflon permettant une filtration à 0,2 µm. Ce filtre est plaqué contre le joint 21 grâce aux ressorts 25, afin d'assurer une étanchéité vis-à-vis de l'extérieur. La face externe du filtre débouche dans un plénum 22 relié à une vanne d'isolement 14. Cette vanne d'isolement est reliée à un évent 5 et à un petit extracteur 16.

Suivant un mode de réalisation préféré de l'invention, l'ensemble constitué par le moule 4, le filtre 6, la réhausse 1 et le piston 2 et la vanne 14 est isolé thermiquement et de ce fait, désinfecté à chaque opération.

L'utilisation d'un gainage de métal et d'un calorifuge peut être nécessaire : un désinfecteur capable de traiter des masses de déchets de 1 kg environ, soit un volume non compacté de 10 litres environ (densité de 0,1), aura pour un diamètre de moule (et donc de piston de compactage) de 160 mm environ, une hauteur de moule et de réhausse de 500 mm. La galette de déchets compactée aura ainsi une hauteur de 50 mm environ (densité de déchets compactés voisine de 1). Assurer une répartition de chaleur sur une telle hauteur dans un matériau comme le métal inoxydable, aussi mauvais conducteur de la chaleur, serait très long et difficile, même avec un très bon calorifuge. Un gainage en aluminium et la mise en place d'un bon calorifuge permettent de résoudre ce problème. De préférence, le gainage est en métal fixé sur le piston.

Selon un mode de réalisation particulier de l'invention, Le moule et le piston sont suffisamment ajustés pour limiter l'extrusion des déchets dans l'interstice. Toutefois, avantageusement, un segment racleur en métal inoxydable, dont le diamètre est très légèrement supérieur à celui du piston, est posé sur la partie basse du piston, avant le joint. Ce segment racleur permet d'éviter l'extrusion vers le joint de déchets, et protège ainsi le joint de la destruction.

Le dispositif selon l'invention est parfaitement étanche, et cette étanchéité est obtenue au moyen de joints astucieusement disposés et, de préférence, de vannes d'isolation 12 et 14. Ces joints peuvent être torriques ou gonflants.

Suivant un mode de réalisation particulier de l'invention, un trou d'évacuation 15 relié à une vanne d'isolement 12 permet, en cas de nécessité, d'évacuer des jus surabondants après désinfection, ou de traiter des déchets liquides.

Lors de la réalisation du procédé selon l'invention, les déchets sont placés dans une zone de compactage, puis soumis à une pression : l'air contenu dans le sac 11 de déchets est aspiré via les trous 7 et le filtre 6 et s'évacue par la vanne 14, en position ouverte. Le piston 2 descendant lentement si le joint 8 est un joint gonflable, de l'air extérieur est même aspiré par l'espace situé entre le moule 4 et le piston 2, interdisant ainsi toute rétrodiffusion de contamination en dehors de la machine. Dans le cas d'une étanchéité réalisée avec un joint torrique, l'étanchéité entre le moule 4 et le piston 2 est effective dès que le joint est en contact avec le moule et l'air contenu dans les déchets ne peut plus s'échapper qu'en passant par le filtre. Un capteur de pression (non représenté) permet de s'assurer que la pression atteinte dans la presse n'atteint pas des valeurs inacceptables, ce qui aurait pour conséquence une ouverture automatique de la vanne 14 ou l'ouverture d'un clapet taré sur le circuit hydraulique du vérin 3, permettant au piston de remonter.

Lorsque le compactage est terminé, dans le cas d'une étanchéité réalisée avec un joint gonflable 8, celui-ci est actionné et la vanne d'isolement 14 est fermée pour rendre étanche la zone de traitement où se trouvent les déchets compactés. Les résistances électriques 9 et 13 sont alors mises en route jusqu'à la température de consigne. La température et la pression à l'intérieur de l'enceinte de traitement sont simultanément contrôlées par un thermocouple ou une sonde platine.

De préférence, les déchets sont préalablement recouverts de polymère, de préférence composite, ou placés dans un sac en polymère, de préférence composite, avant d'être introduits dans la zone de compactage. Un mode de réalisation préféré de l'invention consiste à utiliser un sac en polymère composite, la surface extérieure du sac étant en polypropylène ou en polyamide et la surface intérieure du sac étant en polyéthylène.

L'ensemble constitué par le moule et du piston est thermiquement isolé : du fait de la présence du calorifuge 17 de 10 cm d'épaisseur environ, les pertes thermiques sont très faibles. A titre d'exemple, pour une machine capable de traiter des sacs de déchets de 1 à 2 kg, soit un moule de diamètre intérieur de 160 mm et une hauteur de déchets compactés de 50 mm environ, les pertes thermiques sont estimées à environ 250 W, pour une température de traitement de 180°C.

Le temps de réalisation du procédé selon l'invention est variable et dépend notamment de la température à laquelle l'opérateur souhaite porter le sac 11 de déchets. Par exemple, pour un traitement à 130°C, la durée de montée en température pourra être d'une heure environ et la durée du traitement pourra être d'une heure environ. A 180°C, la durée de montée en température pourra être de deux heures environ et la durée du traitement pourra n'être que de quelques minutes.

Lorsque le temps de traitement requis est atteint, le chauffage est arrêté. On peut alors soit laisser les déchets compactés en place jusqu'à ce qu'ils refroidissent, ce qui, compte-tenu de la bonne isolation thermique mise en place autour du moule et du piston, nécessitera plusieurs heures, améliorant d'autant le traitement, soit le retirer afin de traiter un autre sac de déchets. Dans ce cas, la vanne d'isolement 14 est ouverte progressivement, ce qui permet de détendre l'eau surchauffée contenue généralement dans les déchets (sang, urine, etc ....) et donc de l'évacuer sous forme de vapeur. Cette vaporisation conduit à un refroidissement plus rapide de la galette de déchets compactés.

L'étape suivante consiste à refroidir la zone de compactage jusqu'à une température inférieure à 60° C, à faire revenir cette zone à pression atmosphérique et à évacuer le sac compacté de déchets. Pour ce faire, le vérin 3 est actionné pour remonter le piston 2. Pour le démoulage proprement dit, suivant un mode de réalisation particulier de l'invention, le dispositif selon l'invention comprend deux caractéristiques supplémentaires:
- le moule 4 présente une contre-dépouille ;
- la face du piston 2 qui est en contact avec les déchets compactés est munie d'ergots 10 permettant l'accrochage de la galette de déchets au piston 2 lors de sa remontée.

Après dégagement complet du piston 2 et de la galette de déchets, un petit mouvement de translation de la galette de déchets par rapport au piston, obtenu grâce à la butée 19 située sur le cadre permet de les désolidariser et de faire tomber la galette de déchets dans le chariot 18. On peut ensuite réaliser le traitement d'un autre sac de déchets.

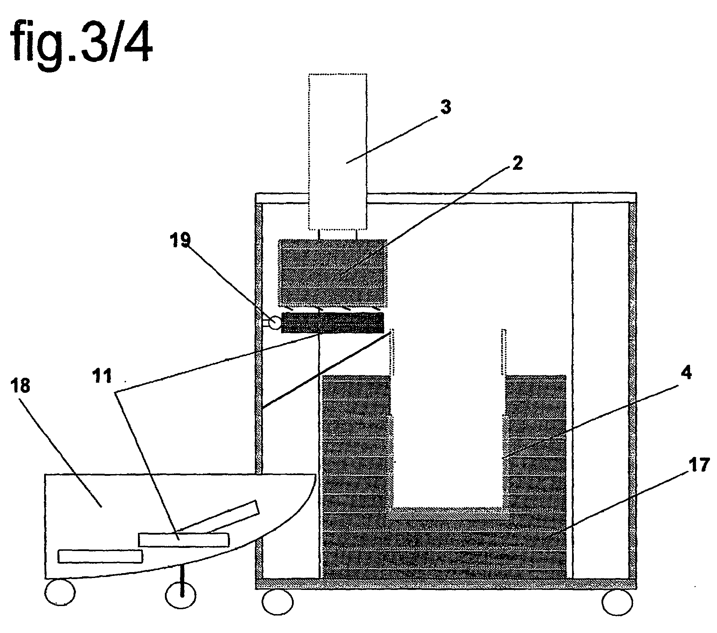

La figure 3 montre la presse en position d'évacuation des déchets compactés : suivant un mode de réalisation préféré de l'invention, le cadre comprend une butée 19, qui facilite l'évacuation des déchets compactés et désinfectés, et qui permet surtout d'évacuer le déchet sans qu'il y ait de contact physique direct avec l'opérateur.

Le dispositif selon l'invention peut également être utilisé pour la désinfection de matériel médical destiné éventuellement à être réutilisé, notamment du matériel métallique, tels que des scalpels, des récipients, etc...

Le fonctionnement de ce dispositif permet de compacter les déchets en assurant le piégeage des particules et vésicules contaminées de l'air enfermé dans les déchets et libéré lors du compactage.

Un avantage de cette étape de compactage est d'augmenter la densité des déchets jusqu'à une valeur proche de sa densité théorique et donc d'améliorer considérablement la conductibilité thermique des déchets et de réduire ainsi la durée nécessaire pour que tous les déchets soient portés de façon homogène à une température suffisante pour la désinfection.

L'utilisation d'un chauffage conventionnel, à savoir la conduction thermique à partir des parois chauffées de la presse, est donc rendue possible, par le compactage des déchets qui voient ainsi leur densité et leur conductibilité thermique atteindre leurs valeurs optimales.

Mais un autre avantage important est également obtenu, à savoir la possibilité de traiter indistinctement des déchets soit entièrement métalliques, soit entièrement exempts d'eau ou présentant des pertes diélectriques nulles, alors que les procédés mettant en oeuvre des sources d'énergie micro-ondes ou haute fréquence ne le permettent pas.

Par ailleurs, l'ensemble de la presse est isolé thermiquement afin que toutes les parties qui auraient pu être contaminées soient portées à la même température et soient donc également désinfectées pendant le traitement.

On voit donc que le dispositif décrit permet une désinfection des déchets à risques biologiques sans contaminer de matériel superflu tel qu'un broyeur et surtout, en s'autodécontaminant à chaque opération.

Par ailleurs, le procédé selon l'invention permet d'éviter le dégagement d'odeurs nauséabondes : le fait de compacter le sac 11 de déchets préalablement au chauffage, et de le traiter en enceinte étanche, réduit considérablement le dégazage et donc les odeurs.

## Revendications

1. Procédé de désinfection thermique de déchets notamment à risques biologiques, **caractérisé en ce que** lesdits déchets sont placés dans une zone de compactage, puis soumis à une pression, les gaz émis par le compactage desdits déchets sont filtrés avant d'être évacués, puis le moule est étanchéifié et un chauffage est appliqué en contrôlant simultanément la température et la pression à l'intérieur du moule, la température dudit chauffage étant incluse entre 100 et 200°C, toutes les pièces ayant été en contact avec les déchets étant également désinfectées.

2. Procédé de désinfection thermique de déchets notamment à risques biologiques selon la revendication 1, **caractérisé en ce que** les déchets sont préalablement recouverts de polymère, de préférence composite, ou placés dans un sac en polymère, de préférence composite, avant d'être introduits dans la zone de compactage.

3. Procédé de désinfection thermique de déchets notamment à risques biologiques selon la revendication 2, **caractérisé en ce que** le sac est en polymère composite, la surface extérieure du sac étant en polypropylène ou en polyamide et la surface intérieure du sac étant en polyéthylène.

4. Procédé de désinfection thermique de déchets notamment à risques biologiques selon l'une quelconque des revendications 1 à 3, **caractérisé en ce que** la température de chauffage est supérieure à 134°C.

5. Procédé de désinfection thermique de déchets notamment à risques biologiques selon l'une quelconque des revendications 1 à 4, **caractérisé en ce que** ledit procédé comprend en outre une étape de démoulage incluant le refroidissement jusqu'à une température inférieure à 60°C de la zone de compactage et du déchet compacté qu'elle contient, le retour de ladite zone à pression atmosphérique et l'évacuation du déchet.

6. Dispositif de désinfection thermique de déchets notamment à risques biologiques pour la mise en oeuvre du procédé selon l'une quelconque des revendications 1 à 5 comprenant un piston 2 muni d'un vérin 3, un moule 4, un filtre 6, un calorifuge 17, un sac 11 rempli de déchets à désinfecter, des moyens de chauffage et des moyens d'étanchéifier ledit dispositif, **caractérisé par le fait que** le piston 2 est prévu apte à refermer de manière étanche le moule 4 au moyen d'un joint torique ou gonflable 8, et que les moyens de chauffage sont constitués de résistances électriques et/ou de varistances dont sont munis le moule 4 et le piston 2.

7. Dispositif de désinfection thermique de déchets notamment à risques biologiques selon la revendication 6, **caractérisé en ce qu'**il contient en outre un capteur de pression et/ou une jauge de contrainte et/ou une vanne d'isolement 14 et/ou un extracteur 16 et/ou un trou d'évacuation 15 relié à une vanne d'isolement 12 et/ou un cadre.

8. Dispositif de désinfection thermique de déchets notamment à risques biologiques selon l'une quelconque des revendications 6 ou 7, **caractérisé en ce que** la face du piston 2 qui est en contact avec les déchets est munie d'ergots 10.

9. Dispositif de désinfection thermique de déchets notamment à risques biologiques selon l'une quelconque des revendications 6 à 8, **caractérisé en ce que** le vérin 3 est un vérin hydraulique ou un vérin mécanique commandé par un moteur électrique.

10. Dispositif de désinfection thermique de déchets notamment à risques biologiques selon l'une quelconque des revendications 6 à 9, **caractérisé par le fait que** les moyens d'étanchéifier le dispositif comportant encore des vannes d'isolement 12 et 14.

11. Dispositif de désinfection thermique de déchets notamment à risques biologiques selon l'une quelconque des revendications 6 à 10, **caractérisé en ce que** le piston 2, le moule 4 et la réhausse de guidage 1 sont totalement ou partiellement élaborés en métal inoxydable ayant une dureté suffisante pour ne pas être rayés ni détériorés lors du compactage d'aiguilles et de scalpels.

12. Dispositif de désinfection thermique de déchets notamment à risques biologiques selon l'une quelconque des revendications 6 à 11, **caractérisé en ce que** le piston 2, le moule 4 et la réhausse de guidage 1 sont totalement ou partiellement élaborés ou recouverts en tout métal à haute conductibilité thermique.

13. Dispositif de désinfection thermique de déchets notamment à risques selon l'une quelconque des revendications 7 a 12, **caractérisé en ce que** le cadre est muni d'une butée 19.

14. Dispositif de désinfection thermique de déchets notamment à risques biologiques selon l'une quelconque des revendications 6 à 13, **caractérisé en ce que** le filtre 6 est placé de manière à ce que la température dudit filtre et de l'espace se trouvant entre les déchets et ledit filtre 6 soit sensiblement égale à la température de la zone de compactage.

15. Dispositif de désinfection thermique de déchets notamment à risques biologiques selon l'une quelconque des revendications 6 à 14, **caractérisé en ce que** le filtre 6 est un filtre à très haute efficacité tel que notamment une membrane minérale et/ou du charbon actif et/ou un feutre en téflon.

16. Dispositif de désinfection thermique de déchets notamment à risques biologiques selon l'une quelconque 6 à 15, **caractérisé en ce que** le capteur de pression et/ou la jauge de contrainte est disposée sur le circuit hydraulique du vérin 3.

## Patentansprüche

1. Verfahren zur Hitzedesinfektion von nämlich biologisch kontaminierten Abfällen, **dadurch gekennzeichnet, daß** die besagten Abfälle in eine Verdichtungszone gebracht sind, dann sind sie einem Druck ausgesetzt, die durch die Verdichtung der besagten Abfälle ausgeschiedenen Gase sind filtriert, bevor sie abgeleitet werden, dann ist die Form abgedichtet und eine Heizung angewendet, während die Temperatur und der Druck im Innern der Form gleichzeitig kontrolliert werden, wobei die Temperatur der besagten Heizung zwischen 100 und 200°C eingeschlossen sei, alle Stücke, die mit den Abfällen in Kontakt waren, werden ebenfalls desinfiziert.

2. Verfahren zur Hitzedesinfektion von nämlich biologisch kontaminierten Abfällen nach Anspruch 1, **dadurch gekennzeichnet, daß** die Abfälle zuerst mit vorzugsweise Verbundpolymer bedeckt oder in einen Sack aus vorzugsweise Verbundpolymer gebracht sind, bevor sie in die Verdichtungszone eingeführt werden.

3. Verfahren zur Hitzedesinfektion von nämlich biologisch kontaminierten Abfällen nach Anspruch 2, **dadurch gekennzeichnet, daß** der Sack aus Verbundpolymer ist, wobei die Außenoberfläche des Sackes aus Polypropylen oder Polyamid sei, und die innere Oberfläche des Sackes aus Polyäthylen ist.

4. Verfahren zur Hitzedesinfektion von nämlich biologisch kontaminierten Abfällen nach irgendeinem der Ansprüche 1 bis 3, **dadurch gekennzeichnet, daß** die Heizungstemperatur höher als 134° C ist.

5. Verfahren zur Hitzedesinfektion von nämlich biologisch kontaminierten Abfällen nach irgendeinem der Ansprüche 1 bis 4, **dadurch gekennzeichnet, daß** das besagte Verfahren außerdem einen Schritt zum Abheben umfaßt, die das Abkühlen bis zu einer Temperatur unter 60°C der Verdichtungszone und des gepreßten Abfalls, den sie enthält, die Rückkehr der besagten Zone auf den atmosphärischen Druck und die Beseitigung des Abfalls einschließt.

6. Vorrichtung zur Hitzedesinfektion von nämlich biologisch kontaminierten Abfällen zum Durchführen des Verfahrens nach irgendeinem der Ansprüche 1 bis 5, umfassend einen Kolben 2, der mit einer Winde 3 ausgestattet ist, eine Form 4, einen Filter 6, einen Wärmeschutz 17, einen mit zu desinfizierenden Abfällen gefüllten Sack 11, Heizungsmittel und Mittel zur Abdichtung der besagten Vorrichtung, **dadurch gekennzeichnet, daß** der Kolben 2 geeignet vorgesehen ist, die Form 4 mit Hilfe einer ringförmigen oder aufblasbaren Dichtung 8 wasserdicht zu schließen, und daß die Heizungsmittel aus elektrischen Widerständen und/oder Varistoren bestehen, mit denen die Form 4 und der Kolben 2 versehen sind.

7. Vorrichtung zur Hitzedesinfektion von nämlich biologisch kontaminierten Abfällen nach Anspruch 6, **dadurch gekennzeichnet, daß** sie außerdem einen Druckgeber und/oder einen Spannungsmesser und/oder einen Isolationsschieber 14 und/oder einen Extraktor 16 und/oder eine Ableitungsöffnung 15, die mit einem Isolationsschieber 12 verbunden ist, und/oder einen Rahmen umfaßt.

8. Vorrichtung zur Hitzedesinfektion von nämlich biologisch kontaminierten Abfällen nach irgendeinem der Ansprüche 6 oder 7, **dadurch gekennzeichnet, daß** die Fläche des Kolbens 2, die mit den Abfällen in Kontakt ist, mit Nasen 10 versehen ist.

9. Vorrichtung zur Hitzedesinfektion von nämlich biologisch kontaminierten Abfällen nach irgendeinem der Ansprüche 6 bis 8, **dadurch gekennzeichnet, daß** die Winde 3 eine hydraulische oder eine mechanische Winde ist, die durch einen Elektromotor gesteuert ist.

10. Vorrichtung zur Hitzedesinfektion von nämlich biologisch kontaminierten Abfällen nach irgendeinem der Ansprüche 6 bis 9, **dadurch gekennzeichnet, daß** die Mittel, um die Vorrichtung abzudichten, noch Isolationsschieber 12 und 14 umfassen.

11. Vorrichtung zur Hitzedesinfektion von nämlich biologisch kontaminierten Abfällen nach irgendeinem der Ansprüche 6 bis 10, **dadurch gekennzeichnet, daß** der Kolben 2, die Form 4 und der Führungsaufsatz 1 gänzlich oder zum Teil aus nichtrostendem Metall ausgearbeitet sind, das eine ausreichende Härte hat, um bei der Verdichtung von Nadeln und Seziermessern weder geritzt, noch beschädigt zu werden.

12. Vorrichtung zur Hitzedesinfektion von nämlich biologisch kontaminierten Abfällen nach irgendeinem der Ansprüche 6 bis 11, **dadurch gekennzeichnet, daß** der Kolben 2, die Form 4 und der Führungsaufsatz 1 gänzlich oder zum Teil mit einem oder aus einem beliebigen Metall mit hoher thermischen Leitfähigkeit ausgearbeitet oder bedeckt sind.

13. Vorrichtung zur Hitzedesinfektion von nämlich biologisch kontaminierten Abfällen nach irgendeinem der Ansprüche 7 bis 12, **dadurch gekennzeichnet, daß** der Rahmen mit einem Anschlag 19 ausgestattet ist.

14. Vorrichtung zur Hitzedesinfektion von nämlich biologisch kontaminierten Abfällen nach irgendeinem der Ansprüche 6 bis 13, **dadurch gekennzeichnet, daß** der Filter 6 so gesetzt wird, daß die Temperatur des besagten Filters und des Raums, der sich zwischen den Abfällen und dem besagten Filter 6 befindet, im wesentlichen gleich sei wie die Temperatur der Verdichtungszone.

15. Vorrichtung zur Hitzedesinfektion von nämlich biologisch kontaminierten Abfällen nach irgendeinem der Ansprüche 6 bis 14, **dadurch gekennzeichnet, daß** der Filter 6 ein Filter mit sehr hoher Wirksamkeit ist, wie insbesondere eine Mineralmembran und/oder Aktivkohle und/oder ein Teflonfilz.

16. Vorrichtung zur Hitzedesinfektion von nämlich biologisch kontaminierten Abfällen nach irgendeinem der Ansprüche 6 bis 15, **dadurch gekennzeichnet, daß** der Druckgeber und/oder der Spannungsmesser auf dem hydraulischen Kreis der Winde 3 angeordnet ist.

## Claims

1. Process for thermal disinfection of viz. biologically contaminated waste, **characterised in that** said waste is placed in a compacting area, then subjected to pressure, the gases released due to compacting are filtered before being evacuated, then the mould is tightly closed and heating is applied while simultaneously controlling the temperature and the pressure inside the mould, the temperature of said heating being comprised between 100 and 200°C, all parts having been in contact with the waste being also disinfected.

2. Process for thermal disinfection of viz. biologically contaminated waste according to claim 1, **characterised in that** the waste is previously coated with polymer, preferably composite polymer, or placed in a bag of polymer, preferably composite polymer, before being placed in the compacting area.

3. Process for thermal disinfection of viz. biologically contaminated waste according to claim 2, **characterised in that** the bag is made of composite polymer, the external surface of the bag being of polypropylene or polyamide, and the internal surface of the bag being of polyethylene.

4. Process for thermal disinfection of viz. biologically contaminated waste according to any of claims 1 to 3, **characterised in that** the heating temperature is higher than 134° C.

5. Process for thermal disinfection of viz. biologically contaminated waste according to any of claims 1 to 4, **characterised in that** said process includes, in addition, a step of removal from the mould, including the cooling down to a temperature lower than 60°C of the compacting area and the compacted waste it contains, the return of said area to atmospheric pressure and the evacuation of the waste.

6. Device for thermal disinfection of viz. biologically contaminated waste for implementing the process according to any of claims 1 to 5, comprising a piston 2 provided with a jack 3, a mould 4, a filter 6, a heat insulator 17, a bag 11 filled with waste to be disinfected, heating means and means for tightening said device, **characterised in that** the piston 2 is designed capable of tightly closing the mould 4 by means of an annular or inflatable seal 8, and **in that** the heating means are formed by electric resistors and/or varistors the mould 4 and the piston 2 are provided with.

7. Device for thermal disinfection of viz. biologically contaminated waste according to claim 6, **characterised in that** it contains, in addition, a pressure sensor and/or a strain gauge and/or an insulating valve 14 and/or an exhauster 16 and/or an evacuation hole 15 connected to an insulating valve 12, and/or a frame.

8. Device for thermal disinfection of viz. biologically contaminated waste according to any of claims 6 or 7, **characterised in that** the surface of the piston 2 that is into contact with the waste is provided with catches 10.

9. Device for thermal disinfection of viz. biologically contaminated waste according to any of claims 6 to 8, **characterised in that** the jack 3 is a hydraulic or mechanical jack controlled by an electric motor.

10. Device for thermal disinfection of viz. biologically contaminated waste according to any of claims 6 to 9, **characterised in that** the means for tightening the device also include insulating valves 12 and 14.

11. Device for thermal disinfection of viz. biologically contaminated waste according to any of claims 6 to 10, **characterised in that** the piston 2, the mould 4 and the guiding collar 1 are fully or partially made of stainless metal having a sufficient hardness to be neither scratched nor deteriorated during the compacting of needles or scalpels.

12. Device for thermal disinfection of viz. biologically contaminated waste according to any of claims 6 to 11, **characterised in that** the piston 2, the mould 4 and the guiding collar 1 are fully or partially made or coated with any metal having high thermal conductivity.

13. Device for thermal disinfection of viz. biologically contaminated waste according to any of claims 7 to 12, **characterised in that** the frame is provided with a stop 19.

14. Device for thermal disinfection of viz. biologically contaminated waste according to any of claims 6 to 13, **characterised in that** the filter 6 is placed so that the temperature of said filter and of the space between the waste and said filter 6 is substantially equal to the temperature of the compacting area.

15. Device for thermal disinfection of viz. biologically contaminated waste according to any of claims 6 to 14, **characterised in that** the filter 6 is a very high efficiency filter, such as viz. a mineral membrane and/or active carbon and/or a Teflon felt.

16. Device for thermal disinfection of viz. biologically contaminated waste according to any of claims 6 to 15, **characterised in that** the pressure sensor and/or the strain gauge is arranged on the hydraulic circuit of the jack 3.
